# EUROPEAN PATENT SPECIFICATION

(11) **EP 1 461 308 B1**
(45) Date of publication and mention of the grant of the patent: **20.12.2006**
(21) Application number: 02804264.6
(22) Date of filing: 27.11.2002
(51) Int. Cl.: C07C 259/06, A61K 31/16, A61P 31/04, C12Q 1/00

(54) **BACTERIAL ENZYME INHIBITORS**
BAKTERIELLE ENZYMINHIBITOREN
INHIBITEURS D'ENZYMES BACTERIENNES

(30) Priority: 04.12.2001 GB 0128959
(43) Date of publication of application: 29.09.2004
(73) Proprietor: De Novo Pharmaceuticals Ltd., Histon, Cambridgeshire CB4 9ZR (GB)
(72) Inventor: GANE, Paul De Novo Pharmaceuticals Limited, Cambridgeshire CB4 9ZR (GB); PORTER, Barry De Novo Pharmaceuticals Limited, Cambridgeshire CB4 9ZR (GB); SAROGLOU, Lydia OSI Pharmaceuticals, Oxford OX4 6LT (GB); WILLEMS, Henriette De Novo Pharmaceuticals Limited, Cambridgeshire CB4 9ZR (GB); WIJKMANS, Jac NV Organon, 5340 BH Oss (NL)
(74) Representative: Walls, Alan James
(86) International application number: PCT/GB2002/005347
(87) International publication number: WO 2003/048114

(56) References cited:
- WO-A-00/61134
- WO-A-01/38561
- WO-A-02/26696
- US-A- 4 122 186
- ZIEGLER, ERICH ET AL: "Synthesis of functionally mixed malonic acid derivatives" MONATSHEFTE FUER CHEMIE (1967), 98(3), 1100-3 , XP002241662

## Description

This invention relates to N-formyl hydroxylamine compounds which inhibit the activity of the bacterial enzyme known as peptide deformylase. Since bacterial growth is known to be inhibited by inhibitors of peptide deformylase, the compounds of the invention are expected to include antibacterial agents.

### Background to the Invention

The compounds of the invention are inhibitors of bacterial polypeptide deformylase (PDF; EC 3.5.1.31).

All ribosome-mediated synthesis of proteins starts with a methionine residue. In prokaryotes, the methionyl moiety carried by the initiator tRNA is N-formylated prior to its incorporation into a polypeptide. Consequently, N-formylmethionine is always present at the N-terminus of a nascent bacterial polypeptide. However, most mature proteins do not retain the N-formyl group or the terminal methionine residue. Deformylation is required prior to methionine removal, since methionine aminopeptidase does not recognise peptides with an N-terminal formylmethionine residue (Solbiati et al., J. Mol. Biol. 290:607-614, 1999). Deformylation is, therefore, a crucial step in bacterial protein biosynthesis and the enzyme responsible, PDF, is essential for normal bacterial growth. The gene encoding PDF (def) is present in all pathogenic bacteria for which sequences are known (Meinnel et al., J. Mol. Biol, 266:939-49, 1997). Although a deformylase homologue has recently been cloned from the mitochondria of human cells (Giglione et. el. EMBO Journal, 19, 5916-5929, 2000) it has not been shown to be functional, and its relevance is unknown. Since a number of currently used antibiotics are known to act on both bacteria and mitochondria, PDF is still considered to be a target for antibacterial chemotherapy (for a review see Giglione et al., Mol Microbiol., 36: 1197-1205,2000).

The isolation and characterisation of PDF has been facilitated by an understanding of the importance of the metal ion in the active site (Groche et al., Biophys. Biochem. Res. Commun., 246:324-6, 1998). The Fe²⁺ form is highly active in vivo but is unstable when isolated due to oxidative degradation (Rajagopalan et al., J. Biol. Chem. 273:22305-10, 1998). The Ni²⁺ form of the enzyme has specific activity comparable with the ferrous enzyme but is oxygen-insensitive (Ragusa et al., J. Mol. Biol. 1998, 280:515-23, 1998). The Zn²⁺ enzyme is also stable but is almost devoid of catalytic activity (Rajagopalan et al., J. Am. Chem. Soc. 119:12418-12419, 1997).

Several X-ray crystal structures and NMR structures of E. coli PDF, with or without bound inhibitors, have been published (Chan et al., Biochemistry 36:13904-9, 1997; Becker et al., Nature Struct. Biol. 5:1053-8, 1998; Becker et al., J. Biol. Chem. 273:11413-6, 1998; Hao et al., Biochemistry, 38:4712-9, 1999; Dardel et al., J. Mol. Biol. 280:501-13, 1998; O'Connell et al., J. Biomol. NMR, 13:311-24, 1999), indicating similarities in active site geometry to metalloproteinases such as thermolysin and the metzincins.

The substrate specificity of PDF has been extensively studied (Ragusa et al., J. Mol. Biol. 289:1445-57, 1999; Hu et al., Biochemistry 38:643-50, 1999; Meinnel et al., Biochemistry, 38:4287-95, 1999). These authors conclude that an unbranched hydrophobic chain is preferred at P1', while a wide variety of P2' substituents are acceptable and an aromatic amide substituent may be advantageous at the P3' position. There have also been reports that small peptidic compounds containing an H-phosphonate (Hu et al., Bioorg. Med. Chem. Lett., 8:2479-82, 1998) or thiol (Meinnel et al., Biochemistry, 38:4287-95, 1999; Huntingdon et al., Biochemistry, 39: 4543-51, 2000; Wei et al, J. Combinatorial Chem., 2: 650-57, 2000) metal binding group are micromolar inhibitors of PDF. Peptide aldehydes such as calpeptin (N-Cbz-Leu-norleucinal) have also been shown to inhibit PDF (Durand et al., Arch. Biochem. Biophys., 367:297-302, 1999). Recently, the naturally occurring hydroxamic acid antibiotic actinonin, for which the target of its antibacterial activity was previously unknown, was shown to be a potent inhibitor of polypeptide deformylase (WO 99/39704, and Chen et al, Biochemistry, 39: 1256-62, 2000). Examples of non-peptidic PDF inhibitors with carboxylic acid (Green et al., Arch. Biochem. Biophys. 375: 355-8, 2000; Jayasekera et al., ibid., 381:313-6, 2000) or hydroxamic acid (Apfel et al., J. Med. Chem., 43: 2324-31, 2000) metal binding groups are also known.

It has been reported that PDF is present in eukaryotic parasites such as Plasmodium falciparum (Meinnel, Parasitology Today, 16: 165-8, 2000). Those authors also found evidence for the presence of PDF in other parasites of humans, such as the kinetoplastid protozoan parasites Trypanosoma brucei and Leishmania major. Based on these findings, it is anticipated that the hydroxamic acid and N-formyl hydroxylamine compounds with which this invention is concerned have antiprotozoal activity, and are useful in the treatment of malaria and other protozoal diseases.

Several patent applications describe antibacterial hydroxamic acid and N-formyl hydroxylamine agents whose activity has been attributed to inhibition of PDF. These publications include our copending International patent applications nos. WO 99/39704, WO 99/59568, WO 00/35440, WO 00/44373, WO 00/58294 and WO 00/61134, as well as WO 01/40198 (Aventis), WO 01/44179 (Versicor), WO 01/44178 (Versicor), and WO 01/38561 (Questcor).

Further, actinonin is a naturally occurring antibacterial agent having a hydroxamic acid group, and certain derivatives of actinonin are also known to have antibacterial activity. (see for example Bouboutou et al, Colloq. INSERM (1989)174 (Forum Pept. 2^{nd}, 1988), 341-4; Lelevre et. al. Pathol. Biol. (1989), 37(1), 43-46; Broughton et. al. J. Chem. Soc. Perkin Trans. 1 (1975) (9), 857-60. Tha antibacterial activity of actinonin has been shown to be due, at least in part, to inhibition of PDF (W/O 99/39704 and other publications)

### Brief Description of the Invention

This invention is based on the discovery of a class of N-formyl hydroxylamine derivatives which are inhibitors of the activity of PDF. Compounds in that class are therefore expected to have antibacterial activity. The class includes novel structures which form part of the invention. Also within the scope of the invention is a method of identifying antibacterial agents from within the class of PDF inhibitors of the invention.

### Detailed Description of the Invention

According to the invention there is provided a compound of formula (I), or a salt or hydrate thereof: 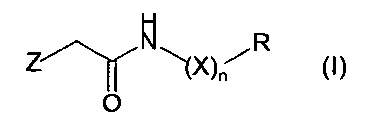 wherein:
Z represents a radical of formula -N(OH)CH(=O) ;
X represents a straight or branched divalent C₁-C₆ alkylene, C₂-C₆ alkenylene, or C₂-C₆ alkynylene radical;
n is 0 or 1; and
R represents an optionally substituted carbocyclic or heterocyclic group, wherein
   a carbocyclic group is a 3-10 membered ring or ring system whose ring atoms are all carbon, and
   a heterocyclic group is a 5-8 membered aromatic or non-aromatic heterocyclic ring containing one or more heteroatoms selected from S, N and O, and optionally fused to a benzyl or second such heterocyclic ring, and
   optionally substituted means substituted with up to four substituents, each of which independently may be (C₁-C₆)alkyl, phenyl, benzyl, (C₁-C₆)alkoxy, benzyloxy, benzyloxy(C₁-C₆)alkyl, phenoxy, phenoxy(C₁-C₆)alkyl, hydroxy, mercapto, (C₁-C₆)alkylthio, amino, halo (including fluoro, chloro, bromo and iodo), trifluoromethyl, cyano, nitro, oxo, - COOH, -CONH₂, -COR^{A}, -COOR^{A}, -NHCOR^{A}, -CONHR^{A}, -NHR^{A}, - NR^{A}R^{B}, or -CONR^{A}R^{B} wherein R^{A} and R^{B} are independently a (C₁-C₆)alkyl group; and in the case where substituted means substituted by phenyl, benzyl, phenyl(C₁-C₆)alkoxy, benzyloxy, benzyloxy(C₁-C₆)alkyl, phenoxy, or phenoxy(C₁-C₆)alkyl, the phenyl ring thereof may itself be substituted with any of (C₁-C₆)alkyl, hydroxy, mercapto, (C₁-C₆)alkylthio, amino, halo (including fluoro, chloro, bromo and iodo), trifluoromethyl, cyano, nitro, oxo, -COOH, -CONH₂, -COR^{A}, -COOR^{A}, -NHCOR^{A}, -CONHR^{A}, -NHR^{A}, -NR^{A}R^{B}, or -CONR^{A}R^{B} wherein R^{A} and R^{B} are independently a (C₁-C₆)alkyl group.

According to a further aspect of the invention, there is provided a method for the identification of antibacterial compounds, comprising screening test compounds for their ability to inhibit PDF *in vitro,* selecting those compounds which exhibit said ability and testing these for their ability to inhibit bacterial growth. The ability to inhibit bacterial growth can be performed using classical plate or broth culture bacterial growth inhibition studies.

As used herein the term "(C₁-C₆)alkyl" means a straight or branched chain alkyl moiety having from 1 to 6 carbon atoms, including for example, methyl, ethyl, n-propyl, isopropyl, n-butyl, isobutyl, sec-butyl, t-butyl, n-pentyl and n-hexyl.

As used herein the term "divalent (C₁-C₆)alkylene radical" means a saturated hydrocarbon chain having from 1 to 6 carbon atoms and two unsatisfied valencies.

As used herein the term "(C₂-C₆)alkenyl" means a straight or branched chain alkenyl moiety having from 2 to 6 carbon atoms having at least one double bond of either E or Z stereochemistry where applicable. The term includes, for example, vinyl, allyl, 1- and 2-butenyl and 2-methyl-2-propenyl.

As used herein the term "divalent (C₂-C₆)alkenylene radical" means a hydrocarbon chain having from 2 to 6 carbon atoms, at least one double bond, and two unsatisfied valencies.

As used herein the term "C₂-C₆ alkynyl" refers to straight chain or branched chain hydrocarbon groups having from two to six carbon atoms and having in addition one triple bond. This term would include for example, ethynyl, 1-propynyl, 1- and 2-butynyl, 2-methyl-2-propynyl, 2-pentynyl, 3-pentynyl, 4-pentynyl, 2-hexynyl, 3-hexynyl, 4-hexynyl and 5-hexynyl.

As used herein the term "divalent (C₂-C₆)alkynylene radical" means a hydrocarbon chain having from 2 to 6 carbon atoms, at least one triple bond, and two unsatisfied valencies.

As used herein the unqualified term "heterocyclyl" or "heterocyclic" includes "heteroaryl" as defined below, and in particular means a 5-8 membered aromatic or non-aromatic heterocyclic ring containing one or more heteroatoms selected from S, N and O, and optionally fused to a benzyl or second heterocyclic ring, and the term includes, for example, pyrrolyl, furyl, thienyl, piperidinyl, imidazolyl, oxazolyl, thiazolyl, thiadiazolyl, thiazepinyl, pyrazolyl, pyridinyl, pyrrolidinyl, pyrimidinyl, morpholinyl, piperazinyl, indolyl, 1,4-dihydroquinolyl, 4H-chromenyl, and benzimidazolyl rings.

As used herein the term "heteroaryl" refers to a 5- or 6- membered aromatic ring containing one or more heteroatoms, and optionally fused to a benzyl or pyridyl ring; and to groups consisting of (a) two such monocyclic or fused rings which are covalently linked; or (b) one such a monocyclic or fused ring covalently linked to an aryl group. Illustrative of such groups are thienyl, furyl, pyrrolyl, imidazolyl, benzimidazolyl, thiazolyl, pyrazolyl, isoxazolyl, isothiazolyl, triazolyl, thiadiazolyl, oxadiazolyl, pyridinyl, pyridazinyl, pyrimidinyl, pyrazinyl, triazinyl, 4-([1,2,3]-thiadiazoly-4-yl)phenyl and 5-isoxazol-3-ylthienyl.

As used herein the unqualified term "carbocyclyl" or "carbocyclic" refers to a 3-10 membered ring or ring system whose ring atoms are all carbon, and includes cycloalkyl, cycloalkenyl and carbocyclic aryl groups.

As used herein the term "cycloalkyl" means a saturated alicyclic moiety having from 3-8 carbon atoms which may be benz-fused and includes, for example, cyclopropyl, cyclobutyl, cyclopentyl, cyclohexyl, cycloheptyl and cyclooctyl.

As used herein the term "cycloalkenyl" means an unsaturated alicyclic moiety having from 5-10 carbon atoms and includes, for example cyclopentenyl, cyclohexenyl, cycloheptenyl and cyclooctenyl. The ring may contain more than one double bond.

As used herein the term "aryl" refers to a mono-or bi-cyclic carbocyclic aromatic group, and to groups consisting of two covalently linked mono-or bi-cyclic carbocyclic aromatic groups. Illustrative of such groups are phenyl, biphenyl and napthyl.

Unless otherwise specified in the context in which it occurs, the term "substituted" as applied to any moiety herein means substituted with up to four substituents, each of which independently may be (C₁-C₆)alkyl, phenyl, benzyl, (C₁-C₆)alkoxy, benzyloxy, benzyloxy(C₁-C₆)alkyl, phenoxy, phenoxy(C₁-C₆)alkyl, hydroxy, mercapto, (C₁-C₆)alkylthio, amino, halo (including fluoro, chloro, bromo and iodo), trifluoromethyl, cyano, nitro, oxo, - COOH, -CONH₂, -COR^{A}, -COOR^{A}, -NHCOR^{A}, -CONHR^{A}, -NHR^{A}, -NR^{A}R^{B}, or -CONR^{A}R^{B} wherein R^{A} and R^{B} are independently a (C₁-C₆)alkyl group. In the case where "substituted" means substituted by phenyl, benzyl, (C₁-C₆)alkoxy, benzyloxy, benzyloxy(C₁-C₆)alkyl, phenoxy, or phenoxy(C₁-C₆)alkyl, the phenyl ring thereof may itself be substituted with any of (C₁-C₆)alkyl, hydroxy, mercapto, (C₁-C₆)alkylthio, amino, halo (including fluoro, chloro, bromo and iodo), trifluoromethyl, cyano, nitro, oxo, -COOH, -CONH₂, -COR^{A}, -COOR^{A}, - NHCOR^{A}, -CONHR^{A}, -NHR^{A}, -NR^{A}R^{B}, or -CONR^{A}R^{B} wherein R^{A} and R^{B} are independently a (C₁-C₆)alkyl group.

It is preferred that when n is 1, X is a C₁-C₃ alkylene radical, more specifically a methylene radical.

R may be, for example, cyclopentyl, cyclohexyl, phenyl or naphthyl. Substituents which may be present in R are those listed above in the definition of "substituted" and include esterified carboxyl, chloro and fluoro, methyl and ethyl, carbamate, and benzyloxymethyl.

Specific compounds of the invention include those of the Examples herein.

Compounds of the invention may be prepared by N formylation (for example using formyl acetic anhydride) of a compound of formula (II) wherein X, R and n are as defined in relation to formula (I), and P is an O- 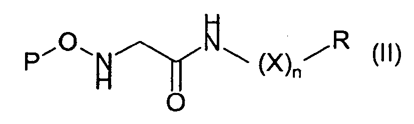 protecting group, followed by removal of the protecting group P. P may be, for example, benzyl, in which case removal may be effected by catalytic hydrogenation.

Compounds of formula (II) may be prepared by coupling a carboxylic acid of formula (III) with an amine of formula (IV) 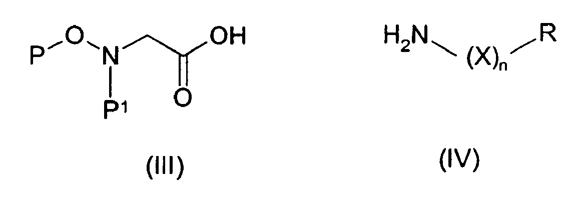 wherein X, R, P and n are as defined in relation to formula (II) and P¹ is an N-protecting group, followed by removal of P¹. P¹ may be, for example, t-butoxycarbonyl, in which case removal of P¹ may be effected by acid hydrolysis. Standard peptide coupling methods may be used for the coupling reaction.

Compounds of formula (III) and (IV) are commercially available, or may be prepared from commercially available precursors using literature methods.

Specific examples of compounds of the invention include those of the Examples herein.

The following abbreviations have been used throughout
- WSCDI: Water soluble carbodiimide
- DMF: Dimethylformamide
- ESMS: Electrospray mass spectroscopy
- HOBt: 1-Hydroxy-7-benzotriazole
- HPLC: High performance liquid chromatography
- LRMS: Low resolution mass spectrometry
- NMR: Nuclear Magnetic Resonance
- RT: Retention time
- THF: Tetrahydrofuran

¹H and ¹³C spectra were recorded using a Bruker DPX 250 spectrometer at 250.1 MHz (62.5 MHz for the ¹³C) and a Bruker AMX 500 spectometer at 500MHz (125 MHz for the ¹³C). Mass spectra were obtained using a Perkin Elmer Sciex API 165. Analytical HPLC was run on a Beckman System Gold, using Waters Symmetry C18 column (50 mm, 4.6 mm) with 20 to 90% solvent B gradient (1.5 ml/min) as the mobile phase. [Solvent A: 0.05% TFA in 10% MeCN 90% water, Solvent B: 0.05% TFA in 10% water 90% MeCN, 5 min gradient time], detection wavelength at 220 or 214 nm. Preparative HPLC was run on a Gilson autoprep instrument using a C18 Waters delta prep-pak cartridge (15µm, 300 A, 25 mm, 10 mm). The method used utilises a constant flow rate (15 ml/min) and solvent A (90% H₂O - 10% acetonitrile) and solvent B (10% H₂O - 90% acetonitrile). The solvent composition is varied as follows: 0-4 mins isocratic 9/1 solvent A / solvent B, 4 to 12 min gradient 9/1 solvent A / solvent B to 1/9 solvent A / solvent B, 12 to 18 min isocratic 1/9 solvent A / solvent B and 18 to 20 min gradient 1/9 solvent A / solvent B to 9/1 solvent A / solvent B. UV detection was at 220 or 214 nm. Reagents were purified and dried where necessary by standard techniques.

### Example 1

### N-Cyclohexyl-2-(formyl-hydroxy-amino)-acetamide

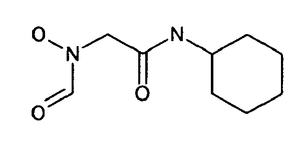

The title compound was prepared as described below:

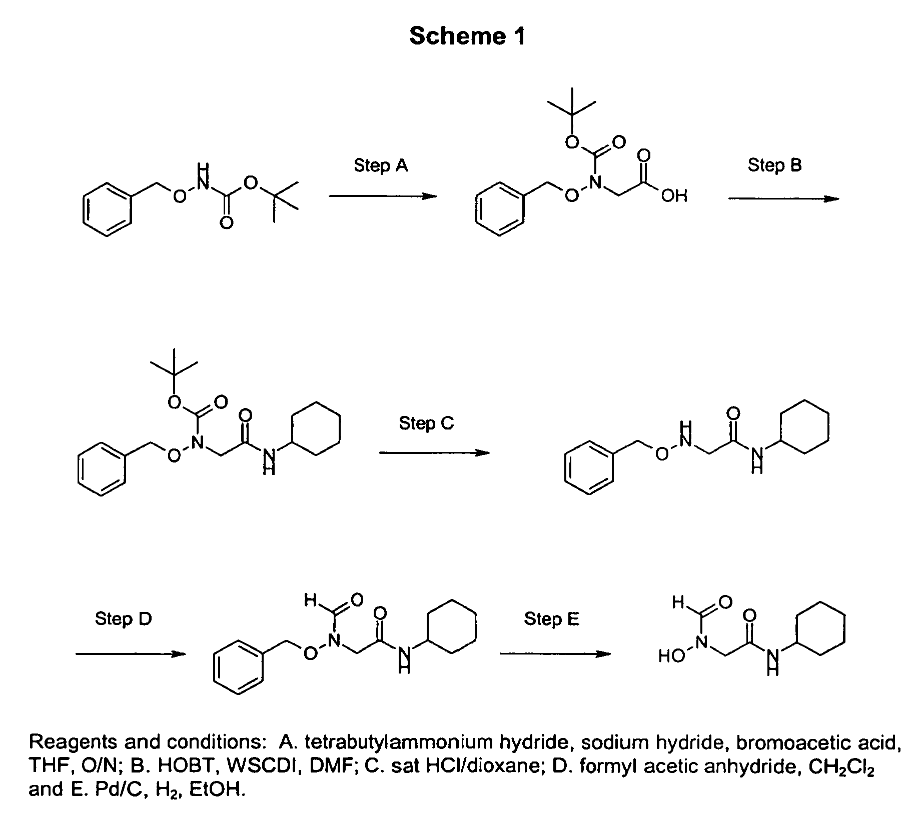

### Step A: N-tert-Butoxycarbonyl-benzyloxyamino-acetic acid

To a solution of *tert*-butyl-*N*-(benzyloxy)-carbamate (2.0 g, 9.0 mmol), bromoacetic acid (1.4 g, 9.9 mmol) and tetrabutyl ammonium iodide (330 mg, 9.9 mmol) in anhydrous THF (80 ml), was added sodium hydride-60% dispersion in oil (0.8 g, 18.8 mmol) under an inert atmosphere at 0°C. The reaction mixture was allowed to warm to room temperature and was stirred overnight at room temperature. The reaction was quenched with water (5 ml) and the solvent was removed under reduced pressure. The residue was dissolved in 1M Na₂CO₃ (200 ml) and was washed with EtOAc (200 ml). The basic aqueous phase was acidified to pH=3 with concentrated hydrochloric acid and the organics were extracted into EtOAc (200 ml). The organic phase was washed with brine (200 ml) and dried over anhydrous magnesium sulphate and the solvent was removed *in vacuo.* The title compound was obtained as a yellow oil (1.5 g, 58%). ¹H-NMR δ (CDCl₃):7.41-7.33 (5H, m), 4.89 (2H, s), 4.10 (2H, s) and 1.50 (9H, s).

### Step B: 2-Benzyloxyamino-N-tert-butoxycarbonyl-N-cyclohexylactamide

To a solution of cyclohexylamine (320 µl, 2.8 mmol), HOBT (280 mg, 2.1 mmol) and WSCDI (390 mg, 2.1 mmol) in DMF (15 ml), was added a solution of *N*-*tert*-butoxycarbonyl-benzyloxyamino-acetic acid (480 mg, 1.7 mmol) in DMF (5 ml). The reaction mixture was stirred overnight. The solvent was removed under reduced pressure and the residue was redissolved in EtOAc (80 ml) and was washed with 1N HCl (80 ml), 1M Na₂CO₃ (80 ml), brine (80 ml) and was dried over anhydrous magnesium sulphate. The solvent was removed under reduced pressure to give the title compound as a white crystalline solid (495 mg, 80%). ¹H-NMR δ (CDCl₃): 7.43-7.31 (5H, m), 5.93 (1H, d), 4.90 (2H, s), 4.01 (2H, s), 3.87-3.68 (1H, m), 1.92-1.79 (2H, m), 1.76-1.62 (3H, m) and 1.43-1.04 (5H, m).

### Step C: 2-Benzyloxyamino-N-cyclohexyl-acetamide

A saturated solution of HCl in dioxane (8 ml) was added to 2-benzyloxyamino-*N*-*tert*-butoxycarbonyl-*N*-cyclohexyl-acetamide (495 µl, 1.4 mmol) and the reaction mixture was stirred for 4 h. The solvent was removed under reduced pressure and the reaction mixture was azeotroped with toluene (2 x 10 ml). The crude mixture was suspended in dichloromethane (15 ml) and polymer supported carbonate resin (780 mg, 2.7 mmol) was added. MeOH (5 ml) was added to aid dissolution of the crude mixture. The resin mixture was shaken for 5 hours. The resin was removed by filtration and the solvent was removed under reduced pressure to give the crude product as a colourless oil (360 mg, 100%). The product was reacted without any purification. ES-MS ions: M+1=263, M+Na=285.

### Step D: 2-(Benzyloxy-formyl-amino)-N-cyclohexyl-acetamide

Formyl acetic anhydride (300 µl, 3.5 mmol) was added to a solution of 2-benzyloxyamino-N-cyclohexyl-acetamide (360 mg, 1.4 mmol) in dichloromethane (20 ml). The reaction mixture was stirred overnight. The solvent was removed under reduced pressure and the crude product was purified by flash chromatography (eluent: EtOAc). The product was obtained as a white solid (278 mg, 70%). ¹H-NMR δ (CDCl₃): 8.16 (1H, s), 7.45-7.32 (5H, m), 5.89 (1 H, s), 4.94 (2H, s), 4.17 (2H, s), 3.88-3.70 (1H, m), 1.95-1.83 (2H, m), 1.76-1.62 (2H, m), 1.61-1.52 (1H, m), 1.48-1.26 (2H, m) and 1.25-1.05 (3H, m).

### Step E: N-Cyclohexyl-2-(formyl-hydroxy-amino)-acetamide

To a solution of 2-(benzyloxy-formyl-amino)-*N* cyclohexyl-acetamide (387 mg, 1.4 mmol) in EtOH (20 ml), was added wet palladium on activated charcoal (60 mg) under an inert atmosphere. Hydrogen gas was bubbled through the reaction mixture for 40 minutes. The reaction mixture was stirred under an inert atmosphere for 30 minutes and the catalyst was filtered. The solvent was removed under reduced pressure to give the title compound as a white solid (185 mg, 96%).

The compounds of Examples 2-8 were prepared by the synthetic route outlined in Scheme 1 and as described in detail for Example 1.

The compounds of Examples 3, 6 and 7 were purified by preparative HPLC.

**Table 1**

| Example | Structure | ES-MS Ions Seen | Proton NMR |
|---|---|---|---|
| 1 | 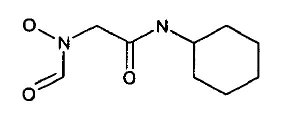 | 2M+1 =401 2M+Na=423 | 1H-NMR; □(CDCl₃), 8.45 (0.6 H, s), 7.97 (0.4H, s), 6.27 (0.4H, d, J=5.0Hz), 6.04 (0.6H, d, J=6.3Hz), 4.31 (1.2H, s), 4.18 (0.8H, s), 3.88-3.65 (1H, m), 1.99-1.81 (2H, m), 1.79. 1.55 (3H, m) and 1.47-1.07 (5H, m). |
| 2 | 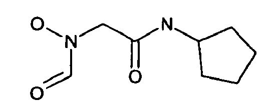 | M+Na=209 M-1=185 | 1H-NMR; (CDCl₃), 8.44 (0.6H. s), 7.96 (0.4H, s), 6.16 (0.4H, bs), 6.10 (0.6H, bs), 4.35-4.10 (3H, m), 2.08-1.88 (2H, m), 1.78-1.52 (4H, m) and 1.75-1.34 (2H, m). |
| 3 | 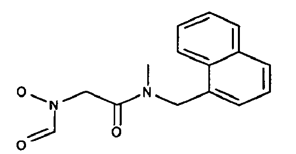 | M+Na=295 | 1H-NMR; (CDCl₃), 8.54 (0.7 H, s), 8.48 (0.3H, s), 8.06-7.78 (3H, m), 7.65-7.18 (4H, m), 5.07 (1.4H, s), 4.98 (0.6H, s), 4.57 (1.4H, s), 4.55 (0.6H, s), 3.09 (0.9H, s) and 2.84 (2.1 H, s). |
| 4 | 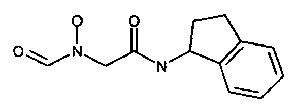 | M+Na=257 | 1H-NMR; ⁻(CH₃OD), 8.40 (0.5 H, s), 8.03 (0.5H, s), 7.31-7.12 (4H, m), 5.42 (1 H, t, J=7.5Hz), 4.88 (1 H, s), 4.28 (1H, s), 3.08-2.78 (2H, m), 2.61-2.42 (1 H, m) and 1.98-1.80 (1 H, m). |
| 5 | 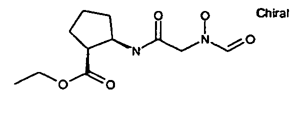 | M+1=259 M+Na=281 | 1H-NMR; ⁻(CH₃OD), 9.11 (0.5 H, s), 8.70 (0.5H, s), 5.29-5.14 (1H, m), 5.02-4.72 (4H, m), 3.83-3.68 (1H, m), 2.82-2.54 (4H, m), 2.52-2.25 (2H, m) and 1.95 (3H, t, J=7.1Hz). |
| 6 | 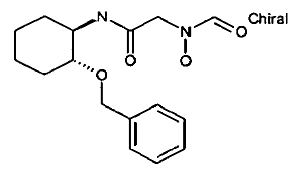 | M+1=307 M+Na=329 | 1H-NMR; (CH₃OD), 8.40 (0.5 H, s), 7.97 (0.5H, s), 7.39-7.19 (5H, m), 4.64-4.47 (2H, m), 4.18 (2H, d, J=20.9Hz), 3.86-3.72 (1 H, m), 2.21-2.12 (1H, m), 2.00-1.82 (1H, m), 1.84 1.62 (2H, m) and 1.42-1.21 (4H, m). |
| 7 | 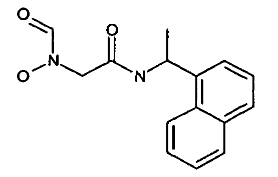 | M+1=273 M+Na=295 | 1H-NMR; (CDCl₃), 8.29 (1 H, s), 8.06-7.88 (1H, m), 7.86-7.70 (2H, m), 7.59-7.32 (4H, m), 7.03 (0.5H, d, J=8.2Hz), 6.74 (0.5H, d, J=7.9Hz), 5.92-5.70 (1H, m), 4.21 (1H, d, J=2.0Hz), 3.91 (1 H, s) and 1.58 (3H, d, J=6.8Hz). |
| 8 | 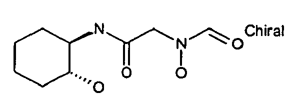 | M+Na=239 M--1=215 | 1H-NMR; (CH₃OD), 8.38 (0.5 H, s), 7.97 (0.5H, s), 4.33-4.07 (2H, m), 3.65-3.52 (1H, m), 3.45-3.30 (1H, m), 2.06-1.86 (2H, m), 1.78-1.62 (2H, m) and 1.42-1.12 (4H, m). |

The compounds of examples 2-8 are named as follows:

### Example 2: N-Cyclopentyl-2-(formyl-hydroxy-amino)-acetamide

### Example 3: 2-(Formyl-hydroxy-amino)-N-methyl-N-naphthalen-1-ylmethylacetamide

### Example 4: 2-(Formyl-hydroxy-amino)-N-indan-1-yl-acetamide

### Example 5: 2-[2-(Formyl-hydroxy-amino)-acetylamino]-cyclopentanecarboxylic acid ethyl ester

### Example 6: N-(1R-2R-Benzyloxy-cyclohexyl)-2-(formyl-hydroxy-amino)-acetamide

### Example 7: 2-(Formyl-hydroxy-amino)-N-(1-naphthalen-1-yl-ethyl)-acetamide

### Example 8: 2-(Formyl-hydroxy-amino)-N-(1 R-2R)-(2-hydroxy-cyclohexyl)-acetamide.

The above compounds were tested for their ability to inhibit the activity of E. coli PDF by the method described in International patent application No. WO99/39704, and were found to inhibit PDF activity, (IC50s <50µM).

## Claims

1. A compound of formula (I), or a salt or hydrate thereof: 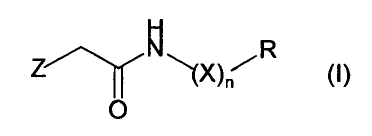 wherein:
Z represents a radical of formula
-N(OH)CH(=O) ;
X represents a straight or branched divalent C₁-C₆ alkylene, C₂-C₆ alkenylene, or C₂-C₆ alkynylene radical;
n is 0 or 1; and
R represents an optionally substituted carbocyclic or heterocyclic group, wherein
a carbocyclic group is a 3-10 membered ring or ring system whose ring atoms are all carbon, and
a heterocyclic group is a 5-8 membered aromatic or non-aromatic heterocyclic ring containing one or more heteroatoms selected from S, N and O, and optionally fused to a benzyl or second such heterocyclic ring, and
optionally substituted means substituted with up to four substituents, each of which independently may be (C₁-C₆)alkyl, phenyl, benzyl, (C₁-C₆)alkoxy, benzyloxy, benzyloxy(C₁-C₆)alkyl, phenoxy, phenoxy(C₁-C₆)alkyl, hydroxy, mercapto, (C₁-C₆)alkylthio, amino, halo (including fluoro, chloro, bromo and iodo), trifluoromethyl, cyano, nitro, oxo, - COOH, -CONH₂, -COR^{A}, -COOR^{A}, -NHCOR^{A}, -CONHR^{A}, -NHR^{A}, - NR^{A}R^{B}, or -CONR^{A}R^{B} wherein R^{A} and R^{B} are independently a (C₁-C₆)alkyl group; and in the case where substituted means substituted by phenyl, benzyl, phenyl(C₁-C₆)alkoxy, benzyloxy, benzyloxy(C₁-C₆)alkyl, phenoxy, or phenoxy(C₁-C₆)alkyl, the phenyl ring thereof may itself be substituted with any of (C₁-C₆)alkyl, hydroxy, mercapto, (C₁-C₆)alkylthio, amino, halo (including fluoro, chloro, bromo and iodo), trifluoromethyl, cyano, nitro, oxo, -COOH, -CONH₂, -COR^{A}, -COOR^{A}, -NHCOR^{A}, -CONHR^{A}, -NHR^{A}, -NR^{A}R^{B}, or -CONR^{A}R^{B} wherein R^{A} and R^{B} are independently a (C₁-C₆)alkyl group.

2. A compound as claimed in claim 1 wherein n is 1 and X is a C₁-C₃ alkylene radical.

3. A compound as claimed in claim 2 wherein X is a methylene radical.

4. A compound as claimed in any of the preceding claims wherein R is optionally substituted cyclopentyl, cyclohexyl, phenyl or naphthyl, wherein optionally substituted has the same meaning as in claim 1

5. A compound as claimed in claim 4 wherein the optional substituent(s) is (are) selected from esterified carboxyl, chloro, fluoro, methyl, ethyl, carbamate, and benzyloxymethyl.

6. A method for the identification of antibacterial compounds, comprising screening test compounds as claimed in any of the preceding claims for their ability to inhibit PDF *in vitro,* selecting those compounds which exhibit said ability and testing these for their ability to inhibit bacterial growth.

## Patentansprüche

1. Verbindung der Formel (I) oder ein Salz oder Hydrat davon: 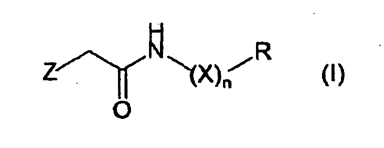 worin:
Z ein Radikal der Formel -N(OH)CH(=O) darstellt;
X ein gerades oder verzweigtes zweiwertiges C₁-C₆-Alkylen-, C₂-C₆-Alkenylen- oder C₂-C₆-Alkinylenradikal darstellt;
n 0 oder 1 ist; und
R eine gegebenenfalls substituierte carbocyclische oder heterocyclische Gruppe darstellt,
worin
die carbocyclische Gruppe ein 3- bis 10-gliedriger(s) Ring oder Ringsystem ist, dessen Ringatome alle Kohlenstoff sind,
die heterocyclische Gruppe ein 5- bis 8-gliedriger aromatischer oder nicht-aromatischer heterocyclischer Ring, enthaltend ein oder mehrere Heteroatome, ausgewählt aus S, N und O, und optional fusioniert mit einem Benzyl- oder einem zweiten solchen heterocyclischen Ring ist, und
gegebenenfalls substituiert mit bis zu 4 Substituenten substituiert bedeutet, von denen jeder unabhängig (C₁-C₆)Alkyl, Phenyl, Benzyl, (C₁-C₆)Alkoxy, Benzyloxy, Benzyloxy(C₁-C₆)alkyl, Phenoxy, Phenoxy(C₁-C₆)alkyl, Hydroxy, Mercapto, (C₁-C₆)Alkylthio, Amino, Halogen (einschließlich Fluor, Chlor, Brom und Iod), Trifluormethyl, Cyano, Nitro, Oxo, -COOH, -CONH₂, -COR^{A}, -COOR^{A}, -NHCOR^{A}, -CONHR^{A}, -NHR^{A}, -NR^{A}R^{B} oder -CONR^{A}R^{B} sein kann, worin R^{A} und R^{B} unabhängig eine (C₁-C₆)Alkylgruppe sind; und in dem Fall, wo substituiert durch Phenyl, Benzyl, Phenyl(C₁-C₆)alkoxy, Benzyloxy, Benzyloxy(C₁-C₆)alkyl, Phenoxy oder Phenoxy(C₁-C₆)alkyl substituiert bedeutet, der Phenylring dann selbst mit einem von (C₁-C₆)Alkyl, Hydroxy, Mercapto, (C₁-C₆)Alkylthio, Amino, Halogen (einschließlich Fluor, Chlor, Brom und Iod), Trifluormethyl, Cyano, Nitro, Oxo, -COOH, -CONH₂, -COR^{A}, -COOR^{A}, -NHCOR^{A}, -CONHR^{A}, -NHR^{A}, -NR^{A}R^{B} oder -CONR^{A}R^{B} substituiert sein kann, worin R^{A} und R^{B} unabhängig eine (C₁-C₆)Alkylgruppe sind.

2. Verbindung gemäß Anspruch 1, worin n 1 und X ein C₁-C₃-Alkylenradikal ist.

3. Verbindung gemäß Anspruch 2, worin X ein Methylenradikal ist.

4. Verbindung gemäß einem der vorangehenden Ansprüche, worin R gegebenenfalls substituiertes Cyclopentyl, Cyclohexyl, Phenyl oder Naphthyl ist, worin gegebenenfalls substituiert dieselbe Bedeutung wie in Anspruch 1 hat.

5. Verbindung gemäß Anspruch 4, worin der/die optional(en) Substituent(en) aus verestertem Carboxyl, Chlor, Fluor, Methyl, Ethyl, Carbamat und Benzyloxymethyl ausgewählt ist/sind.

6. Verfahren zur Identifikation von antibakteriellen Verbindungen, umfassend das Screenen von Testverbindungen gemäß einem der vorangehenden Ansprüche, auf ihre Fähigkeit, PDF in vitro zu inhibieren, Auswählen derjenigen Verbindungen, die diese Fähigkeit zeigen, und Testen dieser auf ihre Fähigkeit, bakterielles Wachstum zu hemmen.

## Revendications

1. Composé de formule (I), ou l'un de ses sels ou hydrates : 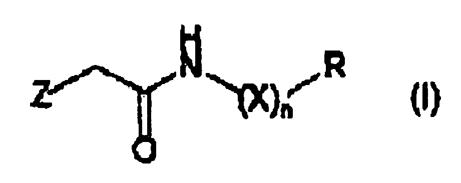 dans laquelle :
Z représente un radical de formule
-N(OH)CH(=O) ;
X représente un radical C₁-C₆ alkylène divalent linéaire ou branché, un C₂-C₆ alkénylène, ou un radical C₂-C₆ alkynylène ;
n est égal à 0 ou 1 ; et
R représente un groupe carboxylique ou hétérocyclique optionnellement substitué, dans lequel
un groupe carboxylique est un cycle ou un système cyclique de 3-10 chaînons dont tous les atomes aux sommets du cycle sont des carbones, et
un groupe hétérocyclique est un hétérocycle aromatique ou non-aromatique de 5-8 chaînons contenant un ou plusieurs hétéroatomes choisis parmi S, N et O, et optionnellement fusionné à un cycle benzyle ou à un second tel hétérocycle, et
optionnellement substitué signifie substitué avec jusqu'à quatre substituants, chacun d'entre eux pouvant être indépendamment un (C₁-C₆)alkyle, un phényle, un benzyle, un (C₁-C₆)alkoxy, un benzyloxy, un benzyloxy(C₁-C₆)alkyle, un phénoxy, un phénoxy(C₁-C₆)alkyle, un hydroxy, un mercapto, un (C₁-C₆)alkylthio, un amino, un halogène (incluant fluoro, chloro, bromo et iodo), un trifluorométhyle, un cyano, un nitro, un oxo, -COOH, - CONH₂, -COR^{A}, -COOR^{A}, -NHCOR^{A}, -CONHR^{A}, -NHR^{A}, - NR^{A}R^{B} ou -CONR^{A}R^{B}, où R^{A} et R^{B} sont indépendamment un groupe (C₁-C₆)alkyle; et dans le cas où substitué signifie substitué par un phényle, un benzyle, un phényl(C₁-C₆)alkoxy, un benzyloxy, un bonzyloxy(C₁-C₆)alkyle, un phénoxy, ou un phénoxy(C₁-C₆)alkyle, ledit cycle phényle peut lui-même être substitué avec l'un des groupes parmi (C₁-C₈)alkyle, hydroxy, mercapto, (C₁-C₆)alkylthio, amino, halogène (incluant fluoro, chloro, bromo et iodo), trifluorométhyle, cyano, nitro, oxo, -COOH, -CONH₂, - COR^{A}, -COOR^{A}, -NHCOR^{A}, -CONHR^{A}, -NHR^{A}, -NR^{A}R^{B}, ou - CONR^{A}R^{B} où R^{A} et R^{B} sont indépendamment un groupe (C₁-C₆)alkyle.

2. Composé tel que revendiqué en revendication 1 dans lequel n est égal à 1 et X est un radical C₁-C₃alkylène.

3. Composé tel que revendiqué en revendication 2 où X est un radical méthylène.

4. Composé tel que revendiqué dans l'une quelconque des revendications précédentes où R est optionnellement substitué par un cyclopentyle, un cyclohexyle, un phényle ou un naphtyle, où optionnellement substitué possède la même signification que dans la revendication 1.

5. Composé tel que revendiqué en revendication 4 dans lequel le(les) substituant(s) optionnel(s) est (sont) choisi(s) parmi les groupes carbonyles estérifiés, chloro, fluoro, méthyle, éthyle, carbamate et benzyloxyméthyle.

6. Méthode pour l'identification de composés antibactériens , comprenant le criblage de composés tests, comme revendiqué dans l'une quelconque des revendications précédentes, pour leur aptitude à inhiber la PDF *in vitro,* la sélection des composés qui possèdent ladite aptitude et le test de leur aptitude à inhiber la croissance bactérienne.
